# EUROPÄISCHE PATENTSCHRIFT

(11) **EP 1 212 281 B1**
(45) Veröffentlichungstag und Bekanntmachung des Hinweises auf die Patenterteilung: **11.08.2004**
(21) Anmeldenummer: 00960571.8
(22) Anmeldetag: 31.08.2000
(51) Int. Cl.: C07C 51/60, C07C 51/64

(54) **VERFAHREN ZUR HERSTELLUNG VON CARBONSÄURECHLORIDEN**
METHOD FOR PRODUCING ACID CHLORIDES
PROCEDE DE FABRICATION DE CHLORURES D'ACIDE

(30) Priorität: 13.09.1999 DE 19943844
(43) Veröffentlichungstag der Anmeldung: 12.06.2002
(73) Patentinhaber: BASF AKTIENGESELLSCHAFT, 67056 Ludwigshafen (DE)
(72) Erfinder: BUSCH, Ralph, 67549 Worms (DE); KNEUPER, Heinz-Josef, 67150 Niederkirchen (DE); WEBER, Theodor, 67063 Ludwigshafen (DE); MÜLLER, Winfried, 68163 Mannheim (DE); STAMM, Armin, 55128 Mainz (DE); HENKELMANN, Jochem, 68165 Mannheim (DE)
(86) Internationale Anmeldenummer: PCT/EP2000/008515
(87) Internationale Veröffentlichungsnummer: WO 2001/019768

(56) Entgegenhaltungen:
- EP-A- 0 296 404
- EP-A- 0 475 137
- DD-A- 153 867

## Beschreibung

Die vorliegende Erfindung betrifft ein Verfahren zur Herstellung von Carbonsäurechloriden durch Umsetzung der entsprechenden Carbonsäuren mit Phosgen oder Thionylchlorid in Gegenwart eines Katalysatoraddukts unter gleichzeitiger und/oder nachträglicher Zufuhr von gasförmigen Chlorwasserstoff, welches zu Carbonsäurechloriden mit niedriger Farbzahl führt.

Carbonsäurechloride sind wichtige Zwischenprodukte bei der Synthese einer Vielzahl chemischer Produkte, insbesondere Pharmazeutika, Kosmetika, Tenside und Papierhilfsmittel. Sie können durch Umsetzung von Carbonsäuren mit Chlorierungsmitteln, wie PCl₃, POCl₃, SOCl₂, SO₂Cl₂ oder COCl₂ hergestellt werden. Von technischer Bedeutung sind vor allem die Umsetzungen mit Thionylchlorid, Phosphortrichlorid und Phosgen.

Bei der Synthese über Phosphortrichlorid wird im allgemeinen ein Reaktand (Carbonsäure oder Phosphortrichlorid) vorgelegt und der andere Reaktand (Phosphortrichlorid oder Carbonsäure) langsam zugeführt. Gegebenenfalls wird die Synthese in einer, mit einem reaktionsinerten Lösungsmittel (z.B. Toluol) verdünnten Lösung durchgeführt. Nach Abtrennung der gebildeten phosphorigen Säure erfolgt in der Regel eine destillative Reinigung des Carbonsäurechlorids. Der Zusatz eines Katalysators ist nicht erforderlich.

EP-A-0 296 404 beschreibt die Reinigung von rohen Carbonsäurechloriden, welche aus der Chlorierung mittels Phosphortrichlorid stammen, bei der die Reaktionsprodukte mit Carbonsäureamid-hydrohalogeniden behandelt werden. Die Carbonsäurechlorid-Rohlösungen der Phosphortrichlorid-Route unterscheiden sich in der Zusammensetzung von denen der Phosgen- bzw. Thionylchloridroute erheblich. So weisen letztere auf:
- (i): Einen wesentlich höheren Gehalt an störenden Nebenkomponenten.
- (ii): Eine unterschiedliche Zusammensetzung der Nebenkomponenten, welche durch die Wahl des Chlorierungs-Agens beeinflußt wird.
- (iii): Ergänzend zur unterschiedlichen Zusammensetzung der Nebenkomponenten, noch die Anwesenheit von Abbau- und/oder Folgeprodukten aus den eingesetzten Katalysator-Addukten.

Die Verwendung von Phosgen oder Thionylchlorid anstelle von Phosphortrichlorid führt in der Regel zu einem höheren Umsatz und einer besseren Selektivität. Beide Chlorierungsmittel haben gegenüber Phosphortrichlorid zudem den Vorteil, daß nur gasförmige Nebenprodukte gebildet werden, die entweder während der Synthese gasförmig entweichen oder durch Strippung mit einem Inertgas nach beendeter Reaktion vollständig ausgetrieben werden können. Desweiteren ist speziell Phosgen ein sehr preiswertes Chlorierungsmittel.

Im Gegensatz zu Phosphortrichlorid als Chlorierungsmittel sind Thionylchlorid und vor allem Phosgen weniger reaktiv. Die Herstellung von Carbonsäurechloriden durch Umsetzung von Carbonsäuren mit Thionylchlorid wird daher zur Erhöhung der Reaktionsgeschwindigkeit vorzugsweise in Gegenwart eines Katalysators durchgeführt. Bei der Herstellung durch Umsetzung mit Phosgen wird stets ein Katalysator eingesetzt. Für beide Chlorierungsmittel geeignete Katalysatorvorstufen sind N,N-disubstituierte Formamide und deren Hydrochloride, aber auch Pyridin oder Harnstoff. Übersichten, betreffend die Chlorierung mittels Thionylchlorid, sind gegeben in M.F. Ansell in S. Patai, "The Chemistry of Acyl Halides", John Wiley and Sons, New York 1972, 35-69 und H.H. Bosshard et al., Helv. Chem. Acta 62 (1959) 1653-1658 sowie S.S. Pizey, Synthetic Reagents, Vol. 1, John Wiley and Sons, New York 1974, ISBN 853120056, 321-557, speziell 333-335. Sowohl nach der Phosgen-Route als auch nach der Thionylchlorid-Route werden N,N-disubstituierte Formamide bevorzugt eingesetzt. Diese setzen sich mit den genannten Chlorierungsmitteln zu den sogenannten Vilsmeier-Salzen um.

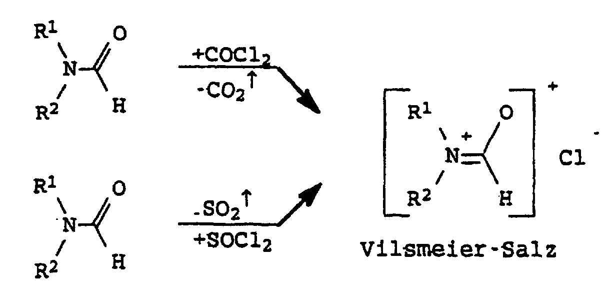

Das Vilsmeier-Salz, das eigentlich reaktive Chlorierungsreagenz, reagiert mit der Carbonsäure oder dem Carbonsäureanhydrid zum Säurechlorid. Dabei wird Formamid-Hydrochlorid zurückgebildet, das wiederum mit Phosgen oder Thionylchlorid zum Vilsmeier-Salz reagieren kann und weitere Katalysatorkreisläufe durchläuft. Die N,N-disubstituierten Formamid-Hydrochloride bzw. deren Vilsmeier-Salze sind jedoch thermisch nicht sehr stabil, so daß es oberhalb von 80 bis 90°C zu Nebenreaktionen kommen kann.

Die bevorzugte Verwendung von N,N-disubstituierten Formamiden als Katalysatorvorstufe für die Phosgenierung von Carbonsäuren geht auch aus EP-A-0 367 050, EP-A-0 452 806, DE-A-4 337 785, EP-A-0 475 137 und EP-A-0 635 473 hervor.

In Bezug auf die Farbzahl wirkt sich bei der Chlorierung von Carbonsäuren mit Phosgen oder Thionylchlorid der Einsatz von Katalysatoren nachteilig aus. Diese werden zwar nach der Chlorierung durch Phasentrennung abgetrennt, können aber in geringen Mengen im Produkt verbleiben und entweder selbst oder als Abbauoder Folgeprodukte zu Gelbfärbungen der Carbonsäurechloride führen. Im allgemeinen werden daher die über Phosgen oder Thionylchlorid hergestellten Carbonsäurechloride destillativ zu weitgehend farblosen Produkten gereinigt. Eine solche Destillation ist nicht nur ein energie- und zeitaufwendiger Vorgang, sondern birgt auch noch eine Reihe weiterer Nachteile. Viele längerkettige Carbonsäurechloride lassen sich nicht ohne partielle Zersetzung destillieren. Weiter ist bekannt, daß durch Zersetzung des im Destillationssumpf noch vorhandenen Katalysators die destillierten Produkte verunreinigt werden können. Größere Mengen an aufgepegeltem Katalysatorrückstand stellen bei der Destillation auch ein Sicherheitsrisiko dar, da in der Hitze die Gefahr einer spontanen Zersetzung besteht.

Eine weitere Möglichkeit zur Reinigung der rohen Carbonsäurechloride ist die Behandlung mit Aktivkohlen. Diese absorptiven Reinigungsschritte sind jedoch technisch aufwendig und zudem nicht immer erfolgreich. Des weiteren fällt kontaminierter Feststoff an, welcher anschließend fachgerecht entsorgt werden muß.

Es bestand daher die Aufgabe, ein verfahren zur Herstellung von Carbonsäurechloriden durch Umsetzung der entsprechenden Carbonsäuren mit Phosgen oder Thionylchlorid zu entwickeln, welches die bekannten Nachteile nicht mehr besitzt und zu Carbonsäurechloriden mit niedriger Farbzahl führt.

Die Aufgabe wurde gelöst durch die Entwicklung eines Verfahrens zur Herstellung von Carbonsäurechloriden durch Umsetzung von Carbonsäuren mit Phosgen in Gegenwart eines Katalysator-Addukts aus einem N,N-disubstituierten Formamid der allgemeinen Formel (I) 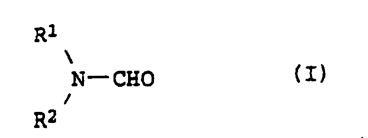 in der R¹ und R² unabhängig voneinander C₁- bis C₄-Alkyl oder R¹ und R² gemeinsam eine C₄- oder C₅-Alkylenkette bedeuten, und Phosgen bei 0 bis 100°C und 0.5 bis 2,0 bar abs. einer Molmenge an N,N-disubstituiertem Formamid (1) von 0.05 bis 2,0 bezogen auf die Molmenge der eingesetzten Carbonsäure und einer Molmenge an Phosgen von 1,0 bis 2,0 bezogen auf die Molmenge an Carbonsäure, das dadurch gekennzeichnet ist, daß man während und/oder nach der Umsetzung gasförmigen Chlorwasserstoff zuführt.

Nach dem erfindungsgemäßen Verfahren können Carbonsäurechloride durch Umsetzung der entsprechenden Carbonsäuren mit Phosgen in hoher Ausbeute und mit niedriger Farbzahl hergestellt werden. Unter niedriger Farbzahl ist dabei eine Farbzahl zu verstehen, welche maximal 50% der Farbzahl nach APHA bzw. bei ungesättigten Carbonsäurechloriden maximal 75% der Iodfarbzahl beträgt, welche bei Ausübung des Verfahrens nach dem Stand der Technik, also ohne die erfinderische Maßnahme, erreicht wird. Die Bestimmungen der Farbzahl nach APHA und der Iodfarbzahl werden in der Norm DIN EN 1557 (März 1997) beschrieben.

Die erfindungsgemäBe Zufuhr des gasförmigen Chlorwasserstoffs kann auf verschiedene Art und Weise erfolgen. So kann der Chlorwasserstoff in Bezug auf die Zufuhr des Chlorierungsmittels Phosgen ausschließlich während dessen Zugabe, während und nach dessen Zugabe oder ausschließlich nach dessen Zugabe zugeführt werden. Bevorzugt wird der gasförmigen Chlorwasserstoff zeitgleich mit der Zugabe des Chlorierungsmittels zudosiert. Die Zufuhr des Chlorwasserstoffs kann bei den drei genannten Varianten kontinuierlich, d.h. ohne Unterbrechung, oder mit einer oder mehreren Unterbrechungen, bis hin zur pulsartigen Dosierung, zugeführt werden. Zudem kann die Zugabegeschwindigkeit des Chlorwasserstoffs innerhalb eines Zugabeintervalls konstant bleiben oder sich erniedrigen oder erhöhen. Im Sinne einer konstanten Reaktionsführung ist es vorteilhaft, den Chlorwasserstoff kontinuierlich zuzuführen, wobei eine Unterbrechung, beispielsweise im Sinne einer nachträglichen Erhöhung der Chlorwasserstoffkonzentration, durchaus noch vorteilhaft sein kann.

Für das erfindungsgemäße Verfahren ist es unerheblich, ob der Chlorwasserstoff an einer Stelle zusammen mit dem Chlorierungsmittel oder an einer anderen Stelle, räumlich getrennt vom Chlorierungsmittel zugegeben wird. Wesentlich ist jedoch eine sehr gute Durchmischung der Reaktionslösung während der Umsetzung mit dem Chlorierungsmittel sowie während der Einleitung des Chlorwasserstoffs und die Anwesenheit der Katalysatorphase während der Chlorwasserstoff-Einleitung. Die Katalysatorphase wird vorteilhafterweise erst nach der vollständigen Zugabe des Chlorwasserstoffs abgetrennt.

Bei der erfindungsgemäßen Herstellung der Carbonsäurechloride wird als Katalysator ein sogenanntes Katalysator-Addukt eingesetzt, welches aus der Umsetzung von Phosgen mit einem N,N-disubstituierten Formamid stammt. Letzteres, welches auch als Katalysatorvorstufe zu bezeichnen ist, ist bestimmt durch die allgemeine Formel (I) 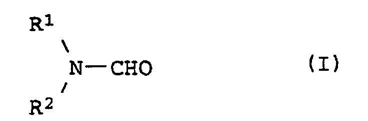 in der R¹ und R² unabhängig voneinander ein C₁- bis C₄-Alkyl, konkret Methyl, Ethyl, Propyl, 1-Mathylethyl, Butyl, 1-Methylpropyl, 2-Methylpropyl und 1,1-Dimethylethyl, oder gemeinsam eine C₄- oder C₅-Alkylenkette, konkret CH₂CH₂CH₂CH₂ oder CH₂CH₂CH₂CH₂CH₂, bedeuten.

Wesentlich ist, daß die gegenseitige Löslichkeit der Carbonsäurechloride und der durch die Chlorwasserstoffzufuhr gebildeten Hydrochloride der N,N-disubstituierten Formamide (I) gering ist und sich zwei isolierbare Phasen ausbilden.

Bevorzugt eingesetzt wird N,N-Dimethylformamid.

Die Bildung des Katalysator-Addukts kann sowohl in dem Apparat erfolgen, in dem die Chlorierung durchgeführt wird, als auch vorgelagert in einem anderen Apparat. Im letztgenannten Fall wird eine bestimmte Menge des N,N-disubstituierten Formamids in einem separaten Apparat vorgelegt, mit gasförmigen Chlorwasserstoff gesättigt und die gewünschte Menge an Phosgen oder Thionylchlorid zugeführt. Anschließend kann die Mischung in den eigentlichen Reaktionsapparat eingefüllt werden. Im erstgenannten Fall wird die beschriebene Prozedur direkt im Reaktionsapparat durchgeführt. Bevorzugt ist das Inkontaktbringen der Carbonsäure mit dem N,N-disubstituiertem Formamid (I) und nachfolgender, gleichzeitiger Zufuhr des Chlorierungsmittels und des Chlorwasserstoffs. Sofern das Verfahren mit Katalysator-Rückführung betrieben wird, wird die Carbonsäure mit dem rückgeführten Katalysator und gegebenenfalls frischem N,N-disubstituierten Formamid (I) in Kontakt gebracht und analog oben beschrieben, anschließend das Chlorierungsmittel und Chlorwasserstoff zugeführt.

Die Menge des einzusetzenden N,N-disubstituierten Formamids (I) ist abhängig von der Art des Chlorierungsmittels. Beim Einsatz von Phosgen setzt man vorteilhaft eine Molmenge an N,N-disubstituiertem Formamid (I) von 0,05 bis 2,0, bevorzugt von 0,1 bis 1,0 und besonders bevorzugt von 0,1 bis 0,6, bezogen auf die Molmenge der eingesetzten Carbonsäure, ein.

Die Umsetzung zwischen der Carbonsäure und Phosgen erfolgt bei Temperaturen von 0 bis 100°C, bevorzugt von 20 bis 80°C, besonders bevorzugt von 20 bis 60°C. Die Umsetzung erfolgt bei einem Druck von 0,5 bis 2,0 bar abs, bevorzugt von 0,8 bis 1,2 bar abs, besonders bevorzugt bei Atmosphärendruck. Als geeignete Reaktionsapparate seien die dem Fachmann bekannten Apparate für Umsetzungen in der flüssig-/flüssig- und gas-/flüssig-Phase, wie beispielsweise Rührkessel oder Rührkesselkaskaden mit entsprechender Gaseinleitungs- und Gasverteilungstechnik zu nennen.

Die während der Umsetzung zum Carbonsäurechlorid insgesamt zugegebene Molmenge an Phosgen beträgt 1,0 bis 2,0, bezogen auf die Molmenge der eingesetzten Carbonsäure. Bevorzugt ist eine Molmenge von 1,0 bis 1,3, bezogen auf die Molmenge der eingesetzten Carbonsäure.

Die beim gasförmigen erfindungsgemäßen Verfahren insgesamt zuzuführende Molmenge an Chlorwasserstoff ist abhängig von der eingesetzten Molmenge an Carbonsäure und liegt vorteilhafterweise im Bereich zwischen 0,2 und 2,0, bezogen auf die Molmenge an eingesetzter Carbonsäure. Bevorzugt ist eine Molmenge von 0,5 bis 1,5, bezogen auf die Molmenge an eingesetzter Carbonsäure. Wie oben bereits ausgeführt, kann beim erfindungsgemäßen Verfahren die Zufuhr an Chlorwasserstoff während und/oder nach der Umsetzung der Carbonsäuren mit Phosgen erfolgen. Die genannte Molmenge an Chlorwasserstoff entspricht der kumulierten Molmenge über das gesamte Verfahren hinweg. Bei kontinuierlicher Fahrweise sind die angegebenen relativen Molmengen auf die Zeiteinheit zu beziehen, wobei in diesem Fall sowohl die Molmenge des frisch hinzugeführten N,N-disubstituierten Formamids (I) als auch die des rückgeführten Katalysators heranzuziehen sind.

Nach der Umsetzung mit dem Chlorierungsmittel kann das Reaktionsgemisch noch eine weitere Zeit intensiv durchmischt werden, wobei je nach Ausführungsform auch noch weiterer Chlorwasserstoff eingeleitet werden kann. Die nachträgliche, intensive Durchmischung wird im allgemeinen für maximal 1 Stunde durchgeführt, kann je nach Reaktionssystem und gewünschter Produktreinheit jedoch auch entfallen. Zudem ist es auch möglich, nach Beendigung der Zugabe des Chlorierungsmittels noch weiteres N,N-disubstituiertes Formamid, bevorzugt unter weiterem Einleiten von Chlorwasserstoff oder als Hydrochlorid, zuzugeben und intensiv zu durchmischen. Dieses kann beispielsweise nach der Chlorierung zugegeben werden, um die Menge an Extraktionsmittel zu erhöhen.

Wesentlich zur Erzielung einer niedrigen Farbzahl der hergestellten Carbonsäurechloride ist die Zusammensetzung der katalysatorhaltigen Phase nach der Umsetzung. Je geringer der Anteil des Katalysator-Addukts ist, desto niedriger ist auch die erzielbare Farbzahl der Carbonsäurechloride. Der molare Anteil des Katalysator-Addukts, bezogen auf die molare Gesamtmenge an N,N-disubstituiertem Formamid (I) plus Katalysator-Addukt, beträgt nach dem erfindungsgemäßen Verfahren vorteilhafterweise weniger als 0,3. Bevorzugt ist ein relativer Anteil von weniger als 0,1, besonders bevorzugt von weniger als 0,05. Der relative Anteil ist über die zugegebene Menge an Chlorierungsmittel und an Chlorwasserstoff einstellbar.

Die Isolierung der Carbonsäurechloride und der katalysatorhaltigen Phase erfolgt vorteilhafterweise durch Phasentrennung. Diese kann sowohl im zuvor verwendeten Reaktionsapparat, sofern dieser hierzu geeignet ist, als auch in einem separaten Apparat durchgeführt werden. Geeignete Apparate sind beispielsweise Rührkessel, Rührkesselkaskaden oder Phasentrenngefäße, wie "Mixer-Settler".
Im allgemeinen haben sich beide Phasen innerhalb von 2 Stunden getrennt. Zur Abtrennung können auch geeignete Filter, wie beispielsweise Koaleszierfilter bekannter Bauart, eingesetzt werden.

Die derart hergestellten Carbonsäurechloride zeigen gegenüber den nach Stand der Technik, ohne die erfindungsgemäße Maßnahme, hergestellten Carbonsäurechloride eine deutlich niedrigere Farbzahl und können nun in der Regel direkt für weitere Synthesestufen eingesetzt werden. Bei Bedarf können sie jedoch auch noch weiteren Behandlungsprozeduren unterzogen werden. Beispielsweise seien genannt die Behandlung mit einem Hydrochlorid eines N,N-disubstituierten Formamids, die Destillation oder die Adsorptivreinigung.

Gemäß einer vorteilhaften Ausführungsform des Verfahrens wird die abgetrennte katalysatorhaltige Phase, enthaltend N,N-disubstituiertes Formamid (I) und Katalysator-Addukt, erneut als Katalysatorvorstufe in der weiteren Synthese eingesetzt. Hierzu führt man die katalysatorhaltige Phase in die Synthesestufe, wie bereits beschrieben, zurück. Es ist von Vorteil, einen Teil der katalysatorhaltige Phase aus dem System auszuschleusen, um eine Aufpegelung unerwünschter Nebenkomponenten zu vermeiden.

Das erfindungsgemäße Verfahren kann sowohl diskontinuierlich als auch kontinuierlich durchgeführt werden.
(a) diskontinuierliche Herstellung:
   Bei der diskontinuierlichen Herstellung wird das Reaktionsgemisch, bestehend aus der Carbonsäure und dem N,N-disubstituierten Formamid (I) bzw. dem Katalysator-Addukt, hergestellt aus Phosgen und dem N,N-disubstituierten Formamid (I), in einem Reaktionsapparat, beispielsweise einem Rührkessel, vorgelegt. Nun wird die gewünschte Menge an flüssigem oder gasförmigem Phosgen sowie parallel dazu die gewünschte Menge an Chlorwasserstoff über einen bestimmten Zeitraum zugegeben. Der Zeitbedarf für die Zugabe des Chlorierungsmittels richtet sich nach der Reaktionsgeschwindigkeit und kann im allgemeinen auf wenige Stunden begrenzt werden. Je nach Ausführungsform endet in der einen Variante die Zufuhr an Chlorwasserstoff mit der Beendigung der Zugabe des Chlorierungsmittels oder wird in einer anderen Variante noch darüber hinaus aufrecht erhalten. Nach Beendigung der Chlorwasserstoffzugabe läßt man die Reaktionslösung im allgemeinen 1 bis 2 Stunden absitzen und trennt die beiden Phasen voneinander. In der Regel befindet sich die Carbonsäurechlorid-enthaltende Phase oben, die katalysatorhaltige Phase unten.
   Es sei explizit darauf hingewiesen, daß in einer dritten Variante mit der erfindungsgemäßen Zufuhr an Chlorwasserstoff auch erst nach Beendigung der Zufuhr des Chlorierungsmittels begonnen werden kann. In diesem Fall würde die Umsetzung mit dem Chlorierungsmittel ohne Zufuhr von Chlorwasserstoff erfolgen.
(b) kontinuierliche Herstellung:
   Für die kontinuierliche Fahrweise geeignete Reaktionsapparate sind beispielsweise Rührkessel, Rührkesselkaskaden oder im Gegenstrom betriebene Reaktionskolonnen. Bei Verwendung eines Rührkessels legt man die Carbonsäure und das N,N-disubstituierte Formamid (I) bzw. das Katalysator-Addukt, hergestellt aus Phosgen und dem N,N-disubstituierten Formamid (I), vor und gibt flüssiges oder gasförmiges Phosgen oder Thionylchlorid sowie parallel dazu die gewünschte Menge an Chlorwasserstoff zu. Nach Einleiten einer der Carbonsäure etwa äquivalenten Menge an Chlorierungsmittel beginnt man gleichzeitig Carbonsäure und N,N-disubstituiertes Formamid (I) bzw. Katalysator-Addukt sowie eine, im wesentlichen der zugeführten Carbonsäure äquimolaren Menge an Phosgen zuzufahren. Des weiteren führt man die gewünschte Menge an Chlorwasserstoff kontinuierlich zu. Eine der zugefahrenen Reaktanden entsprechende Menge des Reaktionsvolumens wird dem Reaktionsapparat, beispielsweise über eine Standhaltung, entnommen und in ein Trenngefäß geleitet.
      Im Trenngefäß kann das Carbonsäurechlorid als obere Phase kontinuierlich entnommen und die katalysatorhaltige, untere Phase kontinuierlich dem Reaktor zurückgeführt werden. Bei der Reaktionsführung ist darauf zu achten, daß das durch die Reaktionsabgase mitgerissene Chlorierungsmittel durch zusätzlich zugeführtes ausgeglichen wird.
   Es sei explizit darauf hingewiesen, daß in einer weiteren Variante des erfindungsgemäßen Verfahrens auch nach Ausschleusung aus dem Reaktionsapparat noch Chlorwasserstoff zugegeben werden kann. Dies kann beispielsweise in einem weiteren, zwischen dem Reaktionsapparat und dem Trenngefäß befindlichen Apparat, beispielsweise einem Rührkessel, erfolgen. Zudem ist es auch möglich, ausschließlich die nachträgliche Chlorwasserstoffzufuhr durchzuführen. In diesem Fall erfolgt die Umsetzung mit dem Chlorierungsmittel ohne Zufuhr von Chlorwasserstoff.

Bevorzugt werden die Carbonsäurechloride nach dem erfindungs· gemäßen Verfahren durch Umsetzung der entsprechenden Carbonsäuren mit Phosgen als Chlorierungsmittel hergestellt.

Nach dem erfindungsgemäßen Verfahren herstellbare Carbonsäurechloride sind beispielsweise solche der allgemeinen Formel (II) 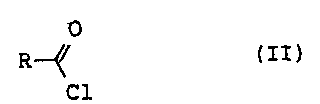 in der R für folgende Reste steht:
C₁- bis C₃₀-Alkyl oder deren aryl- oder cycloalkyl-substituierte Komponenten:
   gesättigter, geradkettiger oder verzweigter Kohlenwasserstoffrest mit 1 bis 30 C-Atomen, bevorzugt Methyl, Ethyl, Propyl, 1-Methylethyl, Butyl, 1-Methylpropyl, 2-Methylpropyl, 1,1-Dimethylethyl, Pentyl, 1-Ethylpropyl, Hexyl, Heptyl, 1-Ethylpentyl, Octyl, 2,4,4-Trimethylpentyl, Nonyl, 1,1-Dimethylheptyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl, Triacontyl, Phenylmethyl, Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl;
C₃- bis C₁₂-Cycloalkyl oder deren aryl- oder cycloalkyl-substituierte Komponenten:
   monocyclischer, gesättigter Kohlenwasserstoffrest mit 3 bis 12 Ring-C-Atomen, bevorzugt Cyclopentyl, Cyclohexyl;
C₂- bis C₃₀-Alkenyl oder deren aryl- oder cycloalkyl-substituierte Komponenten:
   ungesättigter, geradkettiger oder verzweigter Kohlenwasserstoffrest mit 1 bis 30 C-Atomen und 1 bis 5 Doppelbindungen an einer beliebigen Stelle, bevorzugt 2-Propenyl, 3-Butenyl, cis-2-Butenyl, trans-2-Butenyl, cis-8-Heptadecenyl, trans-8-Heptadecenyl, cis,cis-8,11-Heptadecadienyl, cis,cis,cis-8,11,14-Heptadecatrienyl;
C₃- bis C₁₂-Cycloalkenyl oder deren aryl- oder cycloalkyl-substituierte Komponenten:
   monocyclischer, ungesättigter Kohlenwasserstoffrest mit 3 bis 12 Ring-C-Atomen und 1 bis 3 Doppelbindungen an einer beliebigen Stelle, bevorzugt 3-Cyclopentenyl, 2-Cyclohexenyl, 3-Cyclohexenyl, 2,5-Cyclohexadienyl;
C₂- bis C₃₀-Alkinyl oder deren aryl- oder cycloalkyl-substituierte Komponenten:
   ungesättigter, geradkettiger oder verzweigter Kohlenwasserstoffrest mit 1 bis 30 C-Atomen und 1 bis 3 Dreifachbindungen an einer beliebigen Stelle, bevorzugt 3-Butinyl, 4-Pentinyl;
C₄- bis C₃₀-Alkeninyl oder deren aryl- oder cycloalkyl-substituierte Komponenten:
   ungesättigter, geradkettiger oder verzweigter Kohlenwasserstoffrest mit 1 bis 30 C-Atomen, 1 bis 3 Dreifachbindungen und 1 bis 3 Doppelbindungen an einer beliebigen Stelle.

Mit dem erfindungsgemäßen Verfahren können auch Mischungen der genannten Carbonsäurechloride hergestellt werden. Als nicht-limitierende Beispiele seien genannt Mischungen aus C₈- bis C₁₈-Carbonsäurechloriden, welche unter den Trivialnamen "Carbonsäurechlorid", "Talgfettsäurechlorid", "Kokosfettsäurechlorid" und "Ölsäurechlorid" gehandelt werden.

Besonders bevorzugt hergestellt werden nach dem erfindungsgemäßen Verfahren Carbonsäurechloride der allgemeinen Formel (III), in der R für folgende Reste steht:
C₁- bis C₃₀-Alkyl oder deren aryl- oder cycloalkyl-substituierte Komponenten:
   gesättigter, geradkettiger oder verzweigter Kohlenwasserstoffrest mit 1 bis 30 C-Atomen, bevorzugt Methyl, Ethyl, Propyl, 1-Methylethyl, Butyl, 1-Methylpropyl, 2-Methylpropyl, 1,1-Dimethylethyl, Pentyl, 1-Ethylpropyl, Hexyl, Heptyl, 1-Ethylpentyl, Octyl, 2,4,4-Trimethylpentyl, Nonyl, 1,1-Dimethylheptyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl, Phenylmethyl, Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl;
C₂- bis C₃₀-Alkenyl oder deren aryl- oder cycloalkyl-substituierte Komponenten:
   ungesättigter, geradkettiger oder verzweigter Kohlenwasserstoffrest mit 1 bis 30 C-Atomen und 1 bis 5 Doppelbindungen an einer beliebigen Stelle, bevorzugt 2-Propenyl, 3-Butenyl, cis-2-Butenyl, trans-2-Butenyl, cis-8-Heptadecenyl, trans-8-Heptadecenyl, cis,cis-8,11-Heptadecadienyl, cis,cis,cis-8,11,14-Heptadecatrienyl;
   sowie deren Mischungen.

Ganz besonders bevorzugt hergestellt werden nach dem erfindungsgemäßen Verfahren Essigsäurechlorid (R gleich Methyl), Propionsäurechlorid (R gleich Ethyl), Buttersäurechlorid (R gleich Propyl), Valeriansäurechlorid (R gleich Butyl), Isovaleriansäurechlorid (R gleich 2-Methylpropyl), Pivalinsäurechlorid (R gleich 1,1-Dimethylethyl), Capronsäurechlorid (R gleich Pentyl), 2-Ethylbuttersäurechlorid (R gleich 1-Ethylpropyl), Önanthsäurechlorid (R gleich Hexyl), Caprylsäurechlorid (R gleich Heptyl), 2-Ethylhexansäurechlorid (R gleich 1-Ethylpentyl), Pelargonsäurechlorid (R gleich Octyl), Isononansäurechlorid (R gleich 2,4,4-Trimethylpentyl), Caprinsäurechlorid (R gleich Nonyl), Neodecansäurechlorid (R gleich 1,1-Dimethylheptyl), Laurinsäurechlorid (R gleich Undecyl), Myristinsäurechlorid (R gleich Tridecyl), Palmitinsäurechlorid (R gleich Pentadecyl), Stearinsäurechlorid (R gleich Heptadecyl), Ölsäurechlorid (R gleich cis-8-Heptadecenyl), Linolsäurechlorid (R gleich cis,cis-8,11-Heptadecadienyl), Linolensäurechlorid (R gleich cis,cis,cis-8,11,14-Heptadecatrienyl), Arachidinsäurechlorid (R gleich Nonadecyl) und Behensäurechlorid (R gleich Henicosyl)
sowie deren Mischungen.

Die für das erfindungsgemäße Verfahren einzusetzenden Carbonsäuren ergeben sich aus den oben beschriebenen Definitionen für R. Es sei darauf hingewiesen, daß mit dem erfindungsgemäßen Verfahren auch Mischungen der verschiedenen Carbonsäuren chloriert werden können.

In einer allgemeinen Ausführungsform zur diskontinuierlichen Herstellung der Carbonsäurechloride durch Chlorierung mittels Phosgen legt man die gesamte Menge der entsprechenden Carbonsäure in einem Rührkessel vor und dosiert unter Rühren die erforderliche Menge an N,N-disubstituiertem Formamid (I) zu. Das Reaktionssystem wird nun auf die gewünschte Temperatur gebracht und bei Atmosphärendruck unter weiterem intensiven Rühren gasförmiges oder flüssiges Phosgen und gasförmiger Chlorwasserstoff kontinuierlich solange eingeleitet, bis die äquimolare Menge in Bezug auf die eingesetzte Carbonsäure und zusätzlich ein geringer Überschuß an Phosgen in der Reaktionsmischung eingeleitet wurde. Die gebildeten gasförmigen Produkte Kohlendioxid und Chlorwasserstoff werden abgeführt. Bezüglich der Menge des zugeführten Phosgens ist darauf zu achten, daß nach der Umsetzung nur noch eine geringe Konzentration an Katalysator-Addukt vorliegt. Je nach Ausführungsform ist es möglich, die Zufuhr des Chlorwasserstoffs bei Beendigung der Phosgen-Zugabe zu stoppen oder auch noch nach Beendigung der Phosgen-Zugabe aufrecht zu erhalten. Die Gesamtmenge an zugeführtem Chlorwasserstoff sollte jedoch, bezogen auf die Menge an N,N-disubstituiertem Formamid (I), vorteilhafterweise im angegebenen Bereich liegen. Nach Beendigung der Chlorwasserstoff-Zugabe wird die Reaktionslösung noch etwa eine bis zwei Stunden gerührt. Anschließend wird die Durchmischung beendet, so daß sich die beiden Phasen trennen können. Die untere, katalysatorhaltige Phase wird abgetrennt und kann bei weiteren Synthesen wiederverwendet werden. Die verbleibende Carbonsäurechlorid-Phase wird durch Durchleitung von Stickstoff von restlichem Chlorwasserstoff und Kohlendioxid befreit und kann nun für weitere Synthesestufen in der Regel ohne zusätzliche Reinigungsschritte eingesetzt werden.

In einer allgemeinen Ausführungsform zur kontinuierlichen Herstellung der Carbonsäurechloride durch Chlorierung mittels Phosgen werden in einem Rührkessel die Carbonsäure, rückgeführtes Katalysator-Addukt, gegebenenfalls frisches N,N-disubstituiertes Formamid (I), gasförmiges oder flüssiges Phosgen und gasförmiger Chlorwasserstoff unter intensivem Rühren bei der gewünschten Temperatur unter Atmosphärendruck kontinuierlich zugeführt. Die Zugabegeschwindigkeit des Phosgens ist dabei von der der Carbonsäure, die Zugabegeschwindigkeit des Chlorwasserstoffs von der des Katalysator-Addukts bzw. des N,N-disubstituierten Formamids (I) abhängig. Speziell bei der Zufuhr des Phosgens ist darauf zu achten, daB in der entnommenen Lösung nur noch eine geringe Konzentration an Katalysator-Addukt vorliegt. Eine der zugeführten Menge entsprechende Menge wird kontinuierlich aus dem Rührkessel entnommen und einem Trenngefäß zugeführt. Aus diesem wird die katalysatorhaltige Phase, welche sich in'der Regel unten befindet, kontinuierlich abgetrennt und erneut dem Rührkessel zugeführt. Die verbleibende Carbonsäurechlorid-Phase wird dem Trenngefäß entnommen und in einem weiteren Gefäß durch Durchleitung von Stickstoff von restlichem Chlorwasserstoff und Kohlendioxid befreit. Sie kann nun für weitere Synthesestufen in der Regel ohne zusätzliche Reinigungsschritte eingesetzt werden.

Wesentlich bei der oben beschriebenen erfindungsgemäßen Herstellung der Carbonsäurechloride mit niedriger Farbzahl ist der überraschende Effekt, daß sich gerade die farbgebenden Komponenten in der Chlorwasserstoff haltigen Phase des N,N-disubstituierten Formamids (I) erheblich besser lösen als in der Carbonsäurechloridhaltigen Phase.

Das erfindungsgemäße Verfahren führt bereits durch eine, in den Syntheseprozess leicht integrierbare Maßnahme der Chlorwasserstoffzufuhr zu Carbonsäurechloriden mit niedriger Farbzahl, so daß diese in der Regel ohne Destillation, separate Extraktion oder Adsorptivbehandlung für Folgereaktionen eingesetzt werden können. Das erfindungsgemäße Verfahren läßt sich sehr effektiv und wirtschaftlich durchführen. Durch Umgehung der nach Stand der Technik üblichen Destillation werden sowohl Investitions- und Energiekosten eingespart als auch in der Regel eine höhere Ausbeute an gereinigtem-Carbonsäurechlorid erreicht. Für destillationsempfindliche Carbonsäurechloride eröffnet das erfindungsgemäße Verfahren die Möglichkeit einer wirtschaftlichen Synthese im technischen Maßstab.

### Beispiele

### Vergleichsbeispiel 1: Herstellung von Laurinsäurechlorid

4,5 mol Laurinsäure wurden in einer Rührapparatur mit 82,2 g (1,13 mol) N,N-Dimethylformamid versetzt. Die Reaktionslösung wurde unter Rühren auf eine Temperatur von 40 bis 50°C gebracht und unter Atmosphärendruck insgesamt 5,06 mol gasförmiges Phosgen eingeleitet. Nach Beendigung der Phosgen-Zugabe wurden die beiden Phasen voneinander getrennt. Die Katalysatorphase enthielt einen molaren Anteil des Katalysator-Addukts, bezogen auf die molare Menge an N,N-Dimethylformamid plus Katalysator-Addukt, von 0,50. Die Carbonsäurechlorid-Phase enthielt 99,1 Flächen-% Laurinsäurechlorid und 0,15 Flächen-% Laurinsäure. Die Farbzahl betrug 268 APHA.

Durch ein relativ hohes Molverhältnis zwischen dem zugeführten Phosgen und der eingesetzten Laurinsäure wurde ein hoher Umsatz zu Laurinsäurechlorid erreicht. Die Carbonsäurechlorid-haltige Phase zeigt jedoch eine unbefriedigende, hohe Farbzahl.

### Vergleichsbeispiel 2: Herstellung von Pelargonsäurechlorid (Nonansäurechlorid)

2,75 mol Pelargonsäure wurden in einer Rührapparatur mit 100,5 g (1,38 mol) N,N-Dimethylformamid versetzt. Die Reaktionslösung wurde unter Rühren auf eine Temperatur von 20 bis 30°C gebracht und unter Atmosphärendruck insgesamt 2,78 mol gasförmiges Phosgen eingeleitet. Nach Beendigung der Phosgen-Zugabe wurden die beiden Phasen voneinander getrennt. Die Katalysatorphase enthielt einen molaren Anteil des Katalysator-Addukts, bezogen auf die molare Menge an N,N-Dimethylformamid plus Katalysator-Addukt, von < 0,05. Die Carbonsäurechlorid-Phase enthielt 97,1 Flächen-% Pelargonsäurechlorid und 1,9 Flächen-% Pelargonsäureanhydrid. Die Farbzahl betrug 16 APHA.

Durch ein sehr niedriges, nahezu stöchiometrisches Molverhältnis zwischen dem zugeführten Phosgen und der eingesetzten Pelargonsäure wurde nur ein unbefriedigend niedriger Gehalt an Pelargonsäurechlorid im Rohaustrag bei einem zu hohen Gehalt an Pelargonsäureanhydrid erreicht. Allerdings zeigt die Carbonsäurechlorid-haltige Phase eine sehr niedrige Farbzahl.

### Beispiel 3: Herstellung von Pelargonsäurechlorid (Nonansäurechlorid)

2,75 mol Pelargonsäure wurden in einer Rührapparatur mit 100,5 g (1,38 mol) N,N-Dimethylformamid versetzt. Die Reaktionslösung wurde unter Rühren auf eine Temperatur von 20 bis 30°C gebracht und unter Atmosphärendruck insgesamt 2,78 mol gasförmiges Phosgen und gleichzeitig 1,92 mol gasförmiger Chlorwasserstoff eingeleitet. Nach Beendigung der Phosgen- und Chlorwasserstoff-Zugabe wurden die beiden Phasen voneinander getrennt. Die Katalysatorphase enthielt einen molaren Anteil des Katalysator-Addukts, bezogen auf die molare Menge an N,N-Dimethylformamid plus Katalysator-Addukt, von 1%. Die Carbonsäurechlorid-Phase enthielt 98,9 Gew.-% Pelargonsäurechlorid und 0,04 Gew.-% Pelargonsäureanhydrid. Die Farbzahl betrug 18 APHA.

Erst durch die erfindungsgemäße, gleichzeitige Einleitung von Chlorwasserstoff konnte ein hoher Umsatz zu Pelargonsäurechlorid mit einer sehr niedrigen Farbzahl erhalten werden.

### Vergleichsbeispiel 4: Herstellung von Kokosfettsäurechlorid

2,0 mol Kokosfettsäure (Handelsname HK 8-18, Fa. Henkel), welche im wesentlichen aus Laurinsäure und Myristinsäure besteht, wurden in einer Rührapparatur mit 36,6 g (0,5 mol) N,N-Dimethylformamid versetzt. Die Reaktionslösung wurde unter Rühren auf eine Temperatur von 30°C gebracht und unter Atmosphärendruck insgesamt 2,38 mol gasförmiges Phosgen eingeleitet. Nach Beendigung der Phosgen-zugabe wurden die beiden Phasen voneinander getrennt. Die Katalysatorphase enthielt einen molaren Anteil des Katalysator-Addukts, bezogen auf die molare Menge an N,N-Dimethylformamid plus Katalysator-Addukt, von 0,50. Die Carbonsäurechlorid-Phase enthielt 99,6 Gew.-% Kokosfettsäurechlorid und 0,35 Gew.-% Kokosfettsäure. Die Farbzahl betrug 399 APHA.

Durch ein relativ hohes Molverhältnis zwischen dem zugeführten Phosgen und der eingesetzten Kokosfettsäure wurde ein hoher Umsatz zu Kokosfettsäurechlorid erreicht. Die Carbonsäurechlorid-haltige Phase zeigt jedoch eine unbefriedigende, hohe Farbzahl.

### Beispiel 5: Herstellung von Kokosfettsäurechlorid

2,01 mol Kokosfettsäure (Handelsname HK 8-18, Fa. Henkel) wurden in einer Rührapparatur mit 73,1 g (1,0 mol) N,N-Dimethylformamid versetzt. Die Reaktionslösung wurde unter Rühren auf eine Temperatur von 30°C gebracht und unter Atmosphärendruck insgesamt 2,1 mol gasförmiges Phosgen und gleichzeitig 1,04 mol gasförmiger Chlorwasserstoff eingeleitet. Nach Beendigung der Phosgen- und Chlorwasserstoff-Zugabe wurden die beiden Phasen voneinander getrennt. Die Katalysatorphase enthielt einen molaren Anteil des Katalysator-Addukts, bezogen auf die molare Menge an N,N-Dimethylformamid plus Katalysator-Addukt, von < 0,10. Die Carbonsäurechlorid-Phase enthielt 99,5 Gew.-% Kokosfettsäurechlorid und 0,5 Gew.-% Kokosfettsäure. Die Farbzahl betrug 44 APHA.

Erst durch die erfindungsgemäße, gleichzeitige Einleitung von Chlorwasserstoff konnte ein hoher Umsatz zu Kokosfettsäurechlorid mit einer sehr niedrigen Farbzahl erhalten werden.

Die Beispiele zeigen, daß unabhängig von der Art der Carbonsäure, durch gleichzeitiges Einleiten von Chlorwasserstoffgas bei der Umsetzung mit dem Chlorierungsmittel, ein hoher Umsatz zum gewünschten Carbonsäurechlorid mit sehr niedrigen Farbzahlen erreicht wird. Die in den erfindungsgemäßen Beispielen erhaltenen Carbonsäurechloride können ohne weitere Reinigungsschritte in Folgesynthesen eingesetzt werden.

## Patentansprüche

1. Verfahren zur Herstellung von Carbonsäurechloriden durch Umsetzung von Carbonsäuren mit Phosgen in Gegenwart eines Katalysator-Addukts aus einem N,N-disubstituierten Formamid der allgemeinen Formel (I) 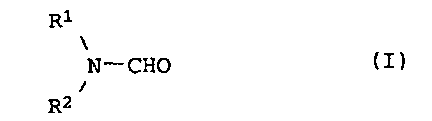 in der R¹ und R² unabhängig voneinander C₁- bis C₄-Alkyl oder R¹ und R² gemeinsam eine C₄- oder C₅-Alkylenkette bedeuten, und Phosgen bei 0 bis 100°C und 0,5 bis 2,0 bar abs, einer Molmenge an N,N-disubstituiertem Formamid (I) von 0,05 bis 2,0, bezogen auf die Molmenge der eingesetzten Carbonsäure und einer Molmenge an Phosgen von 1,0 bis 2,0, bezogen auf die Molmenge an Carbonsäure, **dadurch gekennzeichnet, daß** man während der Umsetzung gasförmigen Chlorwasserstoff zuführt.

2. Verfahren nach Anspruch 1, **dadurch gekennzeichnet, daß** man insgesamt eine molare Menge an Chlorwasserstoff von 0,2 bis 2,0, bezogen auf die molare Menge an eingesetzter Carbonsäure, einsetzt.

3. Verfahren nach den Ansprüchen 1 bis 2, **dadurch gekennzeichnet, daß** der molare Anteil des Katalysator-Addukts aus dem N,N-disubstituierten Formamid (I) und Phosgen, bezogen auf die molare Menge an N,N-disubstituiertem Formamid (I) plus Katalysator-Addukt, nach der Umsetzung weniger als 0,3 beträgt.

4. Verfahren nach den Ansprüchen 1 bis 3, **dadurch gekennzeichnet, daß** der molare Anteil des Katalysator-Addukts aus dem N,N-disubstituierten Formamid (I) und Phosgen, bezogen auf die molare Menge an N,N-disubstituiertem Formamid (I) plus Katalysator-Addukt, nach der Umsetzung weniger als 0,1 beträgt.

5. Verfahren nach den Ansprüchen 1 bis 4, **dadurch gekennzeichnet, daß** man das Carbonsäurechlorid nach der Umsetzung durch Phasentrennung vom Reaktionsgemisch isoliert.

6. Verfahren nach den Ansprüchen 1 bis 5, **dadurch gekennzeichnet, daß** man als N,N-disubstituiertes Formamid (I) N,N-Dimethylformamid einsetzt.

7. Verfahren nach den Ansprüchen 1 bis 6, **dadurch gekennzeichnet, daß** man nach der Umsetzung das N,N-disubstituierte Formamid (I), dessen Hydrochlorid und Katalysator-Addukt abtrennt und erneut als Katalysatorvorstufe in der Carbonsäurechlorid-Synthese einsetzt.

8. Verfahren nach den Ansprüchen 1 bis 7, **dadurch gekennzeichnet, daß** man als Carbonsäurechloride Essigsäurechlorid, Propionsäurechlorid, Buttersäurechlorid, Valeriansäurechlorid, Isovaleriansäurechlorid, Pivalinsäurechlorid, Capronsäurechlorid, 2-Ethylbuttersäurechlorid, Önanthsäurechlorid, Caprylsäurechlorid, 2-Ethylhexansäurechlorid, Pelargonsäurechlorid, Isononansäurechlorid, Caprinsäurechlorid, Neodecansäurechlorid, Laurinsäurechlorid, Myristinsäurechlorid, Palmitinsäurechlorid, Stearinsäurechlorid, Ölsäurechlorid, Linolsäurechlorid, Linolensäurechlorid, Arachidinsäurechlorid und Behensäurechlorid sowie deren Mischungen herstellt.

## Claims

1. A process for the preparation of carbonyl chlorides by reacting carboxylic acids with phosgene in the presence of a catalyst adduct of an N,N-disubstituted formamide of the formula (I) 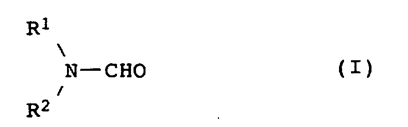 in which R¹ and R² independently of one another are C₁- to C₄-alkyl or R¹ and R² together are a C₄- or C₅-alkylene chain, and phosgene at 0 to 100°C and 0.5 to 2.0 bar abs, a molar amount of N,N-disubstituted formamide (I) of 0.05 to 2.0, based on the molar amount of carboxylic acid employed, and molar amount of phosgene of 1.0 to 2.0, based on the molar amount of carboxylic acid, which comprises introducing gaseous hydrogen chloride during the reaction.

2. A process as claimed in claim 1, wherein, overall, a molar amount of hydrogen chloride of 0.2 to 2.0, based on the molar amount of carboxylic acid employed, is used.

3. A process as claimed in claims 1 and 2, wherein the molar fraction of the catalyst adduct of the N,N-disubstituted formamide (I) and phosgene, based on the molar amount of N,N-disubstituted formamide (I) plus catalyst adduct, is less than 0.3 after the reaction.

4. A process as claimed in claims 1 to 3, wherein the molar fraction of the catalyst adduct of the N,N-disubstituted formamide (I) and phosgene, based on the molar amount of N,N-disubstituted formamide (I) plus catalyst adduct, is less than 0.1 after the reaction.

5. A process as claimed in claims 1 to 4, wherein the carbonyl chloride is isolated from the reaction mixture following the reaction by phase separation.

6. A process as claimed in claims 1 to 5, wherein the N,N-disubstituted formamide (I) used is N,N-dimethylformamide.

7. A process as claimed in claims 1 to 6, wherein, following the reaction, the N,N-disubstituted formamide (I), its hydrochloride and catalyst adduct are separated off and reused as catalyst precursor in the carbonyl chloride synthesis.

8. A process as claimed in claims 1 to 7, wherein the carbonyl chlorides prepared are acetyl chloride, propionyl chloride, butyryl chloride, valeryl chloride, isovaleryl chloride, pivaloyl chloride, caproyl chloride, 2-ethylbutyryl chloride, enanthyl chloride, capryloyl chloride, 2-ethylhexanoyl chloride, pelargonoyl chloride, isononanoyl chloride, capryl chloride, neodecanoyl chloride, lauroyl chloride, myristoyl chloride, palmitoyl chloride, stearoyl chloride, oleoyl chloride, linoleoyl chloride, linolenoyl chloride, arachidoyl chloride and behenoyl chloride, and mixtures thereof.

## Revendications

1. Procédé de préparation de chlorures d'acide carboxylique par réaction d'acides carboxyliques avec du phosgène en présence d'un produit d'addition catalytique constitué d'un formamide N,N-disubstitué de formule générale (I) 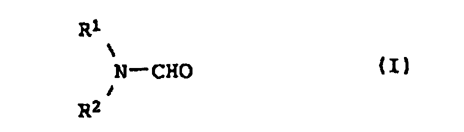 dans laquelle R¹ et R², indépendamment l'un de l'autre, représentent un groupe alkyle en C₁ à C₄ ou R¹ et R², ensemble, représentent une chaîne alkylène en C₄ à C₅, et de phosgène entre 0 et 100°C et entre 0,5 et 2,0 bars abs, une quantité molaire de formamide N,N-disubstitué (I) de 0,05 à 2,0, par rapport à la quantité molaire de l'acide carboxylique utilisée, et une quantité molaire de phosgène de 1,0 à 2,0, par rapport à la quantité d'acide carboxylique, **caractérisé en ce que**, pendant la réaction, on ajoute du chlorure d'hydrogène gazeux.

2. Procédé selon la revendication 1, **caractérisé en ce que** l'on utilise, au total, une quantité molaire de chlorure d'hydrogène allant de 0,2 à 2,0, par rapport à la quantité molaire d'acide carboxylique mis en oeuvre.

3. Procédé selon les revendications 1 à 2, **caractérisé en ce que** la part molaire du produit d'addition catalytique constitué de formamide N,N-disubstitué (I) et de phosgène, par rapport à la quantité molaire de formamide N,N-disubstitué (I) plus le produit d'addition catalytique, après la réaction, est de moins de 0,3.

4. Procédé selon les revendications 1 à 3, **caractérisé en ce que** la part molaire du produit d'addition catalytique constitué de formamide N,N-disubstitué (I) et de phosgène, par rapport à la quantité molaire de formamide N,N-disubstitué (I) plus le produit d'addition catalytique, après la conversion, est de moins de 0,1.

5. Procédé selon les revendications 1 à 4, **caractérisé en ce que** l'on isole du mélange réactionnel le chlorure d'acide carboxylique, après la réaction, par séparation de phase.

6. Procédé selon les revendications 1 à 5, **caractérisé en ce que** l'on utilise, comme formamide N,N-disubstitué (I), du N,N-diméthylformamide.

7. Procédé selon les revendications 1 à 6, **caractérisé en ce que** l'on sépare, après la réaction, le formamide N,N-disubstitué (I), son chlorhydrate et produit d'addition catalytique et on les utilise, de nouveau, à titre de précurseurs catalytiques, dans la synthèse du chlorure d'acide carboxylique.

8. Procédé selon les revendications 1 à 7, **caractérisé en ce que** l'on prépare, à titre de chlorures d'acide carboxylique, du chlorure d'acide acétique, du chlorure d'acide propionique, du chlorure d'acide butyrique, du chlorure d'acide valérianique, du chlorure d'acide isovalérianique, du chlorure d'acide pivalique, du chlorure d'acide caproïque, du chlorure d'acide 2-éthylbutyrique, du chlorure d'acide énanthique, du chlorure d'acide caprylique, du chlorure d'acide 2-éthylhexanoïque, du chlorure d'acide pelargonique, du chlorure d'acide isononanoïque, du chlorure d'acide caprique, du chlorure d'acide néodécanoïque, du chlorure d'acide laurique, du chlorure d'acide myristique, du chlorure d'acide palmitique, du chlorure d'acide stéarique, du chlorure d'acide oléique, du chlorure d'acide linolique, du chlorure d'acide linolénique, du chlorure d'acide arachidique et du chlorure d'acide béhénique et leurs mélanges.
